(19) Europäisches Patentamt
European Patent Office
Office européen des brevets

(11) EP 4 143 153 B1

(12) **FASCICULE DE BREVET EUROPEEN**

(45) Date de publication et mention de la délivrance du brevet:
**20.09.2023 Bulletin 2023/38**

(21) Numéro de dépôt: **21743518.9**

(22) Date de dépôt: **16.07.2021**

(51) Classification Internationale des Brevets (IPC):
***C07C 67/08*** (2006.01) ***C07C 67/307*** (2006.01)
***C07C 69/62*** (2006.01) ***C07C 69/675*** (2006.01)

(52) Classification Coopérative des Brevets (CPC):
(C-Sets disponibles)
**C07C 69/62; C07C 67/08; C07C 67/307** (Cont.)

(86) Numéro de dépôt international:
**PCT/EP2021/070022**

(87) Numéro de publication internationale:
**WO 2022/013440 (20.01.2022 Gazette 2022/03)**

(54) **PROCÉDÉ DE SYNTHÈSE DE DIESTERS D'ACIDE 2-BROMOGLUTARIQUE**

VERFAHREN ZUR SYNTHESE VON 2-BROMGLUTARSÄURE-DIESTERN

METHOD FOR SYNTHESISING DIESTERS OF 2-BROMO GLUTARIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.07.2020 EP 20305823**

(43) Date de publication de la demande:
**08.03.2023 Bulletin 2023/10**

(73) Titulaire: **Guerbet**
**93420 Villepinte (FR)**

(72) Inventeurs:
- **PARATIAN, Jean-Michel**
  **17000 LA ROCHELLE (FR)**
- **CERF, Martine**
  **17870 BREUIL-MAGNÉ (FR)**

(74) Mandataire: **Regimbeau**
**20, rue de Chazelles**
**75847 Paris Cedex 17 (FR)**

(56) Documents cités:
**GB-A- 577 877**

- **FABIO CARNIATO ET AL: "A Chemical Strategy for the Relaxivity Enhancement of GdIII Chelates Anchored on Mesoporous Silica Nanoparticles", CHEMISTRY A EUROPEAN JOURNAL, vol. 16, no. 35, 28 juillet 2010 (2010-07-28), pages 10727-10734, XP055315985, DE ISSN: 0947-6539, DOI: 10.1002/chem.201000499**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



**(Cont. page suivante)**

(52) Classification Coopérative des Brevets (CPC):
(Cont.)

C-Sets
**C07C 67/08, C07C 69/675;**
**C07C 67/307, C07C 69/62**

**Description**

La présente invention a trait à un nouveau procédé de préparation de diesters d'acide 2-bromoglutarique.

Les acides $\alpha$-haloglutariques et leurs esters constituent des « briques » de base utiles en synthèse organique, qui permettent d'intégrer, au sein d'une molécule complexe, un fragment acide $\alpha$-glutarique par une simple réaction de substitution nucléophile.

Le document EP 1 931 673 décrit des nouveaux complexes de gadolinium dérivés de PCTA, ayant des applications en tant qu'agents de contraste dans le domaine de l'imagerie médicale. Les chaînes latérales de certains de ces complexes, et notamment du gadopiclenol, comportent un fragment acide $\alpha$-glutarique. La synthèse du gadopiclenol, sous la forme d'un mélange de l'ensemble de ses stéréoisomères, telle que décrite dans EP 1 931 673 implique l'alkylation du pyclène par le 2-bromoglutarate de diéthyle, aboutissant à un hexaester intermédiaire, qui est ensuite hydrolysé pour conduire à l'hexaacide correspondant, lequel est alors complexé par une source de gadolinium. Le gadopiclenol préparé selon le procédé décrit dans EP 1 931 673 est finalement obtenu par réaction du complexe de gadolinium hexaacide avec le 3-amino-1,2-propanediol.

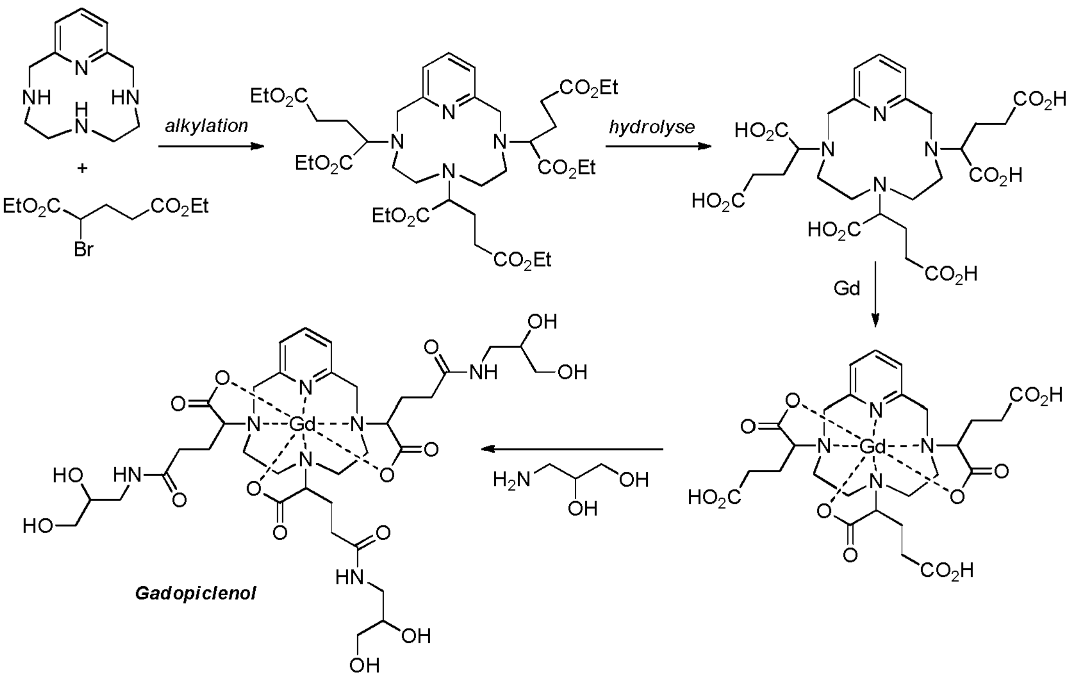


*Synthèse du gadopiclenol*

Or, le 2-bromoglutarate de diéthyle (ci-après BGE) est un composé relativement instable, qui se dégrade dans le temps, sous l'effet de la température ou en présence d'eau. Plus précisément, cet ester $\alpha$-haloglutarique particulier a tendance à s'hydrolyser ou cycliser et donc perdre son atome de brome. Les tentatives de purification du BGE commercial, ou de mise au point de nouvelles voies de synthèse permettant de l'obtenir avec une pureté améliorée, et ainsi d'empêcher sa dégradation, n'ont pas abouti.

Les inventeurs ont donc cherché une alternative au BGE, qui serait plus stable que celui-ci, tout en étant suffisamment réactif pour mener à bien la synthèse du gadopiclenol par exemple. Ainsi, les dérivés chlorés de l'acide glutarique, qui vérifient le critère d'une stabilité améliorée par rapport au BGE, ne constituent pas une option satisfaisante, dans la mesure où ils ne présentent pas une réactivité suffisante. Les dérivés iodés sont quant à eux plus réactifs que leurs analogues bromés, mais aussi plus instables. Les investigations menées par les inventeurs leur ont permis de sélectionner les composés de type 2-bromoglutarate de di-($C_3$-$C_6$)-alkyle comme alternative au BGE, en particulier dans la synthèse du gadopiclenol. Toutefois, les produits commerciaux correspondants ne présentent pas un degré de pureté suffisamment élevé pour être utilisés dans la préparation d'un produit pharmaceutique destiné à une administration humaine, tel que le gadopiclenol.

Il est donc nécessaire de développer un nouveau procédé de préparation de composés de type 2-bromoglutarate de di-($C_3$-$C_6$)-alkyle, qui permette de les obtenir avec une pureté suffisante, et puisse être mis en oeuvre efficacement à l'échelle industrielle.

Or, la synthèse de diesters d'acide 2-bromoglutarique est très peu décrite dans la littérature. Le brevet CS

209266 B1, délivré en 1983, est, à la connaissance des inventeurs, le seul document qui évoque la préparation de ces composés. Il porte de manière générale sur un procédé de préparation d'un acide $\alpha$-haloglutarique ou de l'un de ses diesters alkyliques de formule $R'O_2CCH_2CH_2CH(X)CO_2R''$, dans laquelle R' et R" sont des groupements ($C_1$-$C_5$)alkyle et X correspond à un atome de brome ou de chlore, l'accent étant toutefois nettement mis sur la préparation des dérivés chlorés. Ces derniers sont obtenus par chloration du diester d'acide glutarique en présence d'un catalyseur à base d'antimoine. Si ce document affirme que la méthode décrite permet de réaliser la mono-chloration en position $\alpha$ avec une meilleure sélectivité que les procédés de l'art antérieur, il n'en demeure pas moins que des quantités non négligeables de dérivés $\beta$-chlorés ou $\alpha$-di- et trichlorés sont formées, la sélectivité vis-à-vis du produit $\alpha$-monochloré variant entre 63,16 % et 86,1 % selon la nature du catalyseur à base d'antimoine, le taux de conversion de l'ester de départ oscillant quant à lui entre 86,10 % et 98,9 %. Il est à noter que les conditions pour lesquelles le taux de conversion du produit de départ est le plus élevé ne permettent d'obtenir le produit $\alpha$-monochloré qu'avec une sélectivité de 79 %. Par ailleurs, l'utilisation d'antimoine, extrêmement toxique, constitue un autre inconvénient majeur de ce procédé. Chemistry a European Journal, vol. 16, no. 35, pages 10727-10734 décrit un composé (S)-2-bromopentanedioïque di-tert-butylester. GB 577 877 A divulgue un procédé de préparation d'acides et d'esters dicarboxyliques monobromés.

La présente invention concerne donc un procédé de préparation du diester d'acide 2-bromoglutarique de formule (I) suivante :

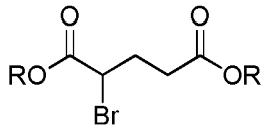

(I)

dans laquelle R représente un groupe ($C_3$-$C_6$)alkyle, comprenant les étapes suivantes :

(b) formation du diester d'acide 2-hydroxyglutarique de formule (II)

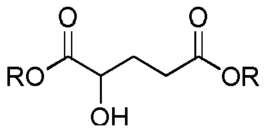

(II)

par réaction de l'acide butyrolactone de formule (BA)

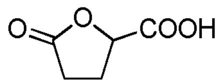

(BA)

avec l'alcool de formule ROH, en présence d'un acide tel que l'acide sulfurique ; et (c) bromation du diester d'acide 2-hydroxyglutarique de formule (II), par bullage d'acide bromhydrique gazeux, conduisant au diester d'acide 2-bromoglutarique de formule (I).

Par groupement « ($C_3$-$C_6$)alkyle », on entend, au sens de la présente invention, une chaîne hydrocarbonée saturée, linéaire ou ramifiée, comportant 3 à 6 atomes de carbone. A titre d'exemple, on peut citer les groupes propyle, isopropyle, *n*-butyle, isobutyle, *sec*-butyle, *tert*-butyle, pentyle ou encore hexyle, en particulier le groupe *n*-butyle, également appelé butyle. L'acide butyrolactone de formule (BA) est également appelé carboxy-$\gamma$-butyrolactone.

Selon un mode de réalisation préféré, R correspond à un groupement butyle, et le procédé selon l'invention permet de préparer le 2-bromoglutarate de dibutyle de formule (BGB), également appelé 2-bromo-1,5-pentanedioate de dibutyle (n° CAS : 104867-13-2).

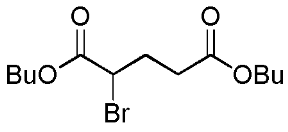

(BGB)

Dans un mode de réalisation particulier, le procédé selon l'invention comprend une première étape **(a)** de formation de l'acide butyrolactone de formule (BA) par réaction de l'acide L-glutamique avec le nitrite de sodium en solution aqueuse.

**Etape (a)**

Cette première étape consiste en la formation de l'acide butyrolactone (BA) à partir de l'acide L-glutamique, matière première couramment disponible, selon une réaction bien connue de l'homme de l'art.

L'acide L-glutamique est introduit dans de l'eau, le rapport de la masse d'eau utilisée sur la masse introduite en acide L-glutamique étant typiquement supérieur à 1, notamment supérieur à 1,5, typiquement égal à 2. La masse volumique de l'eau étant égale à 1 g/mL, dans la suite de la description, un tel rapport masse d'eau (ou par analogie de tout autre solvant ou solution) sur la masse d'un soluté sera désigné par l'expression « équivalent volumique » ou son abréviation « éq. vol. ».

Préférentiellement, l'eau employée dans le procédé est une eau de qualité au moins égale à celle de l'eau désionisée, permettant d'éviter la formation d'impuretés, telle qu'une impureté $\alpha$-chlorée. En particulier, il peut s'agir d'eau désionisée ou d'eau pour injection (eau ppi).

La solution aqueuse résultante est alors typiquement chauffée sous agitation, à une température avantageusement comprise entre 40°C et 70°C, en particulier entre 45°C et 65 °C, de préférence entre 50°C et 60°C, notamment à 55°C.

Une solution aqueuse de nitrite de sodium est ensuite ajoutée progressivement à la solution d'acide L-glutamique, de préférence sous agitation et en maintenant la température précédemment établie.

Ladite solution aqueuse de nitrite de sodium est telle que la quantité d'eau utilisée représente par exemple entre 0,8 et 5,0 éq. vol., notamment entre 1,0 et 3,0 éq. vol., typiquement 2 éq. vol. par rapport à la masse de nitrite de sodium qu'elle contient.

Le nitrite de sodium peut notamment être introduit dans la solution d'acide L-glutamique en léger excès par rapport aux proportions stoechiométriques. Le rapport de la quantité de matière introduite en nitrite de sodium sur la quantité de matière initialement introduite en acide L-glutamique est alors supérieur à 1, mais typiquement inférieur à 1,5, notamment inférieur à 1,3, avantageusement inférieur à 1,2. Autrement dit, la quantité de nitrite de sodium introduite est supérieure à 1 équivalent molaire (éq. mol.), mais typiquement inférieure à 1,5 éq. mol., notamment inférieure à 1,3 éq. mol., avantageusement inférieure à 1,2 éq. mol., par rapport à la quantité d'acide L-glutamique initialement introduite, qui correspond quant à elle à 1 équivalent molaire.

Le mélange réactionnel comprenant le nitrite de sodium et l'acide L-glutamique est ensuite typiquement maintenu sous agitation à une température avantageusement comprise entre 40° C et 70° C, en particulier entre 45 °C et 65 °C, de préférence entre 50° C et 60° C, notamment à 55°C, pendant une durée permettant de solubiliser les différents composés présents en solution, typiquement comprise entre 2h et 10h, de préférence entre 2h et 5h.

Il est ensuite refroidi à une température avantageusement comprise entre 10°C et 45°C, préférentiellement entre 10°C et 35 °C, en particulier entre 15 ° C et 30° C, de préférence entre 20°C et 25°C, puis neutralisé par ajout d'une solution acide, par exemple d'acide chlorhydrique, de préférence à 33% m/m, la quantité de matière introduite en acide chlorhydrique étant alors proche de, typiquement égale Ainsi, la quantité de matière introduite en acide chlorhydrique est supérieure à 1 équivalent molaire (éq. mol.), mais typiquement inférieure à 1,5 éq. mol., notamment inférieure à 1,3 éq. mol., avantageusement inférieure à 1,2 éq. mol., par rapport à la quantité d'acide L-glutamique initialement introduite.

Le mélange réactionnel ainsi neutralisé est ensuite typiquement concentré sous vide, en augmentant progressivement la température, jusqu'à une valeur supérieure à 50°C, par exemple de 60°C.

Dans la suite de la description, par l'expression « sous vide », on entend désigner une pression comprise entre 10 et 500 mbar, notamment entre 10 et 350 mbar, de préférence entre 10 et 150 mbar, en particulier entre 50 et 100 mbar, la température étant précisée le cas échéant.

Concernant l'opération de concentration sous vide permettant d'obtenir, à l'issue de l'étape **(a),** l'acide butyrolactone (BA) brut, celle-ci est typiquement conduite à une pression inférieure à 100 mbar en augmentant graduellement la température jusqu'à atteindre 60°C.

De manière préférentielle, les étapes **(a)** et **(b)** sont effectuées selon un mode de réalisation monotope (ou

« one-pot » en anglais), c'est-à-dire sans étape intermédiaire d'isolement ou de purification.

**Etape (b)**

L'étape **(b)** vise à former le diester d'acide 2-hydroxyglutarique de formule (II)

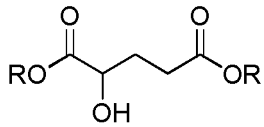

(II)

par réaction de l'acide butyrolactone de formule (BA) avec l'alcool de formule ROH.

Dans un mode de réalisation préféré, l'acide butyrolactone (BA) est issu de l'étape **(a)** précédemment décrite, et n'a pas été purifié avant d'être engagé dans l'étape **(b).**

Au cours de cette étape se produisent de manière concomittante ou successive l'ouverture de la lactone et la formation des deux fonctions esters -C(O)OR.

L'alcool ROH est de préférence introduit en excès par rapport à l'acide butyrolactone. Ainsi, la quantité de ROH introduite est de préférence supérieure ou égale à 2 équivalents molaires (éq. mol.), notamment supérieure ou égale à 4 éq. mol., avantageusement comprise entre 2 et 10 éq. mol., en particulier entre 4 et 10 éq. mol., typiquement égale à 5 éq. mol., par rapport à la quantité d'acide butyrolactone initialement introduite. A noter que lorsque l'acide butyrolactone est issu de l'étape **(a),** la quantité de ROH introduite est exprimée par rapport à la quantité d'acide L-glutamique initialement introduite, le nombre d'éq. mol. indiqué restant inchangé.

Dans un mode de réalisation préféré, l'étape **(b)** est réalisée en présence de l'acétate de formule $CH_3COOR$. La quantité d'acétate introduite est alors typiquement comprise entre à 0,1 et 0,7 éq. mol., notamment entre 0,2 et 0,5 éq. mol., avantageusement entre 0,3 et 0,4 éq. mol., par rapport à la quantité d'alcool ROH introduite.

La formation des deux fonctions esters -C(O)OR, qui intervient au cours de l'étape **(b),** peut être avantageusement réalisée en catalyse acide. Ainsi, l'étape **(b)** est de préférence réalisée en présence d'une quantité catalytique d'acide, par exemple d'acide sulfurique. Dans de telles conditions, la lactone est susceptible de s'ouvrir pour former l'acide 2-hydroxyglutarique de formule (HG), l'estérification de ses fonctions acide carboxylique par l'alcool ROH intervenant de manière subséquente.

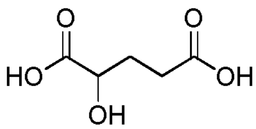

(HG)

Ainsi, l'étape (b) du procédé selon l'invention comprend également la formation du diester d'acide 2-hydroxyglutarique de formule (II) par réaction de l'acide 2-hydroxyglutarique de formule (HG) avec l'alcool de formule ROH.

A l'inverse, l'acide butyrolactone (BA) peut subir une première réaction d'estérification avant ouverture du cycle, pour former la lactone estérifiée de formule (BR) suivante.

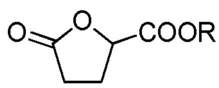

(BR)

Ainsi, l'étape **(b)** du procédé selon l'invention comprend également la formation du diester d'acide 2-hydroxyglutarique de formule (II) par réaction de la lactone estérifiée de formule (BR) avec l'alcool de formule ROH.

Comme cela apparaîtra clairement à l'homme du métier, le procédé selon l'invention peut être mis en oeuvre en utilisant comme produit de départ pour l'étape **(b)** l'acide 2-hydroxyglutarique de formule (HG) ou la lactone estérifiée de formule (BR) comme alternative à l'acide butyrolactone (BA).

Dans un mode de réalisation préféré, l'eau présente dans le mélange réactionnel de l'étape **(b),** typiquement formée *in situ,* est éliminée par distillation sous vide, jusqu'à l'obtention d'un milieu réactionnel ayant une fraction massique

en eau inférieure à 2 % p/p, de préférence inférieure à 1,5 % p/p, avantageusement inférieure à 0,8 % p/p.

Ledit mélange réactionnel de l'étape **(b)** comprend en outre notamment l'acide butyrolactone de formule (BA), l'alcool de formule ROH, et avantageusement de l'acétate de formule $CH_3COOR$.

Avantageusement, la distillation sous vide est une distillation azéotropique sous vide.

Par « distillation azéotropique sous vide », on entend, au sens de la présente invention, la distillation d'un mélange azéotropique réalisée à une pression comprise entre 10 et 500 mbar, notamment entre 10 et 350 mbar, de préférence entre 10 et 150 mbar, en particulier entre 50 et 100 mbar, qui permet d'éliminer l'un des constituants du mélange azéotrope.

En l'espèce, le mélange azéotropique est un mélange azéotropique ternaire eau/ROH/$CH_3COOR$, et la distillation azéotropique sous vide permet d'éliminer l'eau du mélange.

Selon un mode de réalisation préféré, R correspond à un groupement butyle (Bu), et le mélange azéotrope est un mélange ternaire eau/BuOH/$CH_3COOBu$. Cet azéotrope est caractérisé par un point d'ébullition compris entre 85 et 95°C, plus précisément entre 87°C et 93°C, encore plus précisément entre 89°C et 91,5°C, typiquement égal à 89,4°C, à pression atmosphérique.

La distillation azéotropique sous vide du mélange ternaire eau/BuOH/$CH_3COOBu$ est typiquement réalisée à une pression comprise entre 10 et 500 mbar, notamment entre 10 et 350 mbar, de préférence entre 10 et 150 mbar, en particulier entre 50 et 125 mbar, et à une température comprise entre 20°C et 100°C, notamment entre 30°C et 70°C.

Dans un mode de réalisation préféré, à l'issue de l'étape **(b),** le milieu réactionnel est refroidi à une température inférieure à 15 °C, notamment inférieure à 10°C, en particulier comprise entre 0°C et 5°C.

Cette étape de refroidissement intervient typiquement après la distillation sous vide, qui est de préférence une distillation azéotropique sous vide du mélange réactionnel, telle que décrite précédemment.

Dans un mode de réalisation préféré, à l'issue de l'étape **(b)** le milieu réactionnel refroidi est laissé à décanter après ajout d'eau de façon à obtenir la formation d'une phase organique et d'une phase aqueuse distinctes, ladite phase aqueuse étant ensuite éliminée.

Cette opération est avantageusement réalisée plusieurs fois, typiquement entre 2 et 5 fois, en particulier 3 fois.

La quantité d'eau ajoutée représente par exemple entre 0,1 et 2 éq. vol. par rapport à la masse d'acide butyrolactone initialement employée. A noter que lorsque l'acide butyrolactone est issu de l'étape **(a),** la quantité d'eau ajoutée est exprimée par rapport à la masse d'acide L-glutamique initialement introduite, le nombre d'éq. vol. indiqué restant inchangé.

Lorsque l'opération d'ajout d'eau / décantation est réalisée plusieurs fois, la quantité d'eau ajoutée lors de la 1ère occurrence est typiquement comprise entre 1 et 2 éq. vol, et la quantité d'eau ajoutée lors des occurrences suivantes est typiquement comprise entre 0,1 et 0,5 éq. vol.

Le milieu réactionnel obtenu à l'issue de l'étape **(b),** de préférence la phase organique récupérée après la ou les opérations d'ajout d'eau / décantation, comprend typiquement le diester d'acide 2-hydroxyglutarique de formule (II) comme espèce majoritaire, ainsi que la lactone estérifiée de formule (BR), en solution dans l'alcool ROH, avantageusement en mélange avec l'acétate de formule $CH_3COOR$.

Selon un mode de réalisation préféré, R correspond à un groupement butyle (Bu), et le milieu réactionnel obtenu à l'issue de l'étape **(b),** de préférence la phase organique récupérée après la ou les opérations d'ajout d'eau / décantation, comprend typiquement le 2-hydroxyglutarate de dibutyle de formule (HGB) comme espèce majoritaire, ainsi que l'ester butylique de l'acide butyrolactone de formule (BBE), en solution dans le butanol BuOH, avantageusement en mélange avec l'acétate de butyle $CH_3COOBu$.

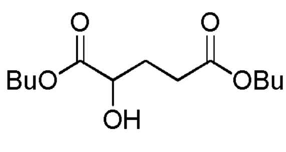

(HGB)

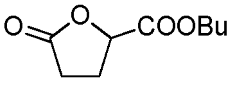

(BBE)

Dans un mode de réalisation préféré, le milieu réactionnel obtenu à l'issue de l'étape **(b),** de préférence la phase organique récupérée après la ou les opérations d'ajout d'eau / décantation, est ensuite déshydraté par distillation sous vide, à une pression comprise entre 10 et 500 mbar, notamment entre 10 et 350 mbar, de préférence entre 10 et

150 mbar, en particulier entre 50 et 100 mbar.

Avantageusement, il s'agit d'une distillation azéotropique sous vide du mélange eau/ROH/$CH_3COOR$, en particulier eau/BuOH/$CH_3COOBu$, et la distillation azéotropique sous vide permet d'éliminer l'eau du mélange.

Dans un mode de réalisation préféré, le milieu réactionnel obtenu à l'issue de l'étape **(b)**, de préférence la phase organique récupérée après la ou les opérations d'ajout d'eau / décantation, avantageusement déshydraté par distillation sous vide, à une pression comprise entre 10 et 500 mbar, notamment entre 10 et 350 mbar, de préférence entre 10 et 150 mbar, en particulier entre 50 et 100 mbar, est ensuite concentré sous vide avant d'être soumis à l'étape (c), de façon à éliminer une partie du mélange ROH/$CH_3COOR$, en particulier BuOH/$CH_3COOBu$.

La quantité dudit mélange éliminé représente typiquement entre 1 et 2 éq. vol. par rapport à la masse d'acide butyrolactone initialement employée ou par rapport à la masse d'acide L-glutamique initialement introduite lorsque l'acide butyrolactone est issu de l'étape **(a).**

Dans la suite de la description, les opérations de refroidissement, d'ajout d'eau / décantation, déshydratation par distillation sous vide et/ou concentration sous vide précédemment décrites sont considérées comme faisait partie intégrante de l'étape **(b)** lorsqu'elles sont réalisées.

De manière préférentielle, les étapes **(b)** et **(c)** sont effectuées selon un mode de réalisation monotope (ou « one-pot » en anglais), c'est-à-dire sans étape intermédiaire d'isolement ou de purification.

**Etape (c)**

L'étape (c) vise à former le diester d'acide 2-bromoglutarique de formule (I) par bromation du diester d'acide 2-hydroxyglutarique de formule (II) obtenu lors de l'étape **(b).**

Comme cela apparaîtra clairement à l'homme du métier au vu de la description détaillée de l'étape **(b),** le milieu réactionnel obtenu à l'issue de l'étape **(b)** comprend typiquement le diester d'acide 2-hydroxyglutarique de formule (II) comme espèce majoritaire, ainsi que la lactone estérifiée de formule (BR), en solution dans l'alcool ROH, avantageusement en mélange avec l'acétate de formule $CH_3COOR$.

Dès lors, bien que l'étape (c) vise à former le diester d'acide 2-bromoglutarique de formule (I) par bromation du diester d'acide 2-hydroxyglutarique de formule (II), la formation du diester d'acide 2-hydroxyglutarique de formule (II) par réaction de la lactone estérifiée de formule (BR) avec l'alcool ROH peut typiquement se poursuivre au cours de cette étape, l'ouverture de la lactone étant facilitée par l'introduction de l'agent de bromation dans le milieu réactionnel.

L'étape **(c)** débute typiquement par l'établissement de la température du milieu réactionnel à une valeur comprise entre 5°C et 40°C, avantageusement entre 10°C et 30°C, en particulier à 20°C.

L'acide bromhydrique gazeux est ensuite progressivement introduit dans le milieu réactionnel par bullage.

Dans un mode de réalisation préféré, la quantité acide bromhydrique gazeux introduite est alors typiquement comprise entre 1 et 1,5 éq. mol., notamment entre 1,2 et 1,3 éq. mol., par rapport à la quantité d'acide butyrolactone engagée dans l'étape **(b)** ou par rapport à la quantité d'acide L-glutamique initialement introduite à l'étape **(a).**

Dans un mode de réalisation préféré, le milieu réactionnel obtenu après introduction de l'acide bromhydrique gazeux est ensuite déshydraté par distillation sous vide, à une pression typiquement comprise entre 10 et 500 mbar, notamment entre 10 et 350 mbar, de préférence entre 10 et 150 mbar, en particulier entre 50 et 100 mbar, pendant une durée typiquement comprise entre 3h et 8h, avantageusement entre 5h et 7h.

Avantageusement, la distillation sous vide est une distillation azéotropique sous vide du mélange eau/ROH/$CH_3COOR$, en particulier eau/BuOH/$CH_3COOBu$. Une telle distillation azéotropique sous vide permet alors d'éliminer l'eau du mélange.

La température du milieu réactionnel est ensuite typiquement rétablie à une valeur comprise entre 5°C et 40°C, avantageusement entre 10° et 30°C, en particulier à 20°C.

Dans un mode de réalisation préféré, le cycle d'opérations précédemment décrit, à savoir l'établissement de la température du milieu réactionnel à une valeur comprise entre 5°C et 40°C, l'introduction de l'acide bromhydrique gazeux dans une quantité typiquement comprise entre à 1 et 1,5 éq. mol par rapport à la quantité d'acide butyrolactone engagée dans l'étape **(b)** ou par rapport à la quantité d'acide L-glutamique initialement introduite à l'étape **(a),** la distillation sous vide pendant une durée comprise entre 3h et 8h de façon à retirer de l'eau du milieu réactionnel, et le rétablissement de la température du milieu réactionnel à une valeur comprise entre 5°C et 40°C, est répété de 3 à 8 fois, de préférence de 4 à 6 fois.

Le milieu réactionnel obtenu à l'issue de l'étape **(c)** comprend typiquement le diester d'acide 2-bromoglutarique de formule (I) en solution dans l'alcool ROH, avantageusement en mélange avec l'acétate de formule $CH_3COOR$.

Selon un mode de réalisation préféré, R correspond à un groupement butyle et le milieu réactionnel obtenu à l'issue de l'étape **(c)** comprend typiquement le 2-bromoglutarate de dibutyle de formule (BGB) en solution dans le butanol BuOH, avantageusement en mélange avec l'acétate de de butyle $CH_3COOBu$.

Alternativement, l'acide bromhydrique peut être formé *in situ* lors de la distillation azéotropique. Dans cette alternative, un sel de brome, typiquement NaBr ou KBr, est introduit dans le milieu réactionnel à la place de l'acide

bromhydrique gazeux, et un acide fort, tel que de l'acide sulfurique concentré (> 96 %), est ensuite ajouté.

L'homme du métier saura adapter les étapes de traitement subséquentes du procédé en conséquence, en particulier de sorte à éliminer les sels présents, par exemple des sels de sulfate, dans le cadre de ce mode de réalisation alternatif.

Etapes (d) - (e)

Dans un mode de réalisation préféré du procédé selon l'invention, le mélange réactionnel obtenu à l'issue de l'étape (c) est soumis aux étapes additionnelles suivantes :

(d) introduction du mélange réactionnel obtenu à l'issue de l'étape (c) dans une solution aqueuse basique, la solution obtenue ayant un pH typiquement compris entre 7,5 et 9,5 ;
(e) décantation de la solution obtenue à l'étape (d), de façon à obtenir la formation d'une phase organique et d'une phase aqueuse distinctes, ladite phase aqueuse étant ensuite éliminée ;
**(f)** concentration sous vide de la phase organique obtenue à l'étape **(e),** jusqu'à atteindre une température comprise entre 65 °C et 75 °C puis séchage sous vide ;
**(g)** optionnellement filtration,

et récupération du diester d'acide 2-bromoglutarique de formule (I).

Dans un mode de réalisation alternatif, les étapes **(d)** et **(e)** ne sont pas effectuées et l'étape **(f)** est réalisée directement sur le mélange réactionnel obtenu à l'issue de l'étape **(c),** et est optionnellement suivie d'une étape de filtration **(g).**

L'étape **(d)** peut notamment être réalisée par addition du mélange réactionnel obtenu à l'issue de l'étape **(c)** dans une solution aqueuse d'ion bicarbonate également appelé hydrogénocarbonate, les quantités du bicarbonate de potassium ou de sodium et d'eau employées pouvant être déterminées par l'homme du métier de sorte à obtenir à l'issue de l'étape **(d)** une solution ayant un pH typiquement compris entre 7,5 et 9,5 , en particulier entre 8 et 9, et à pouvoir opérer une séparation entre une phase aqueuse et organique lors de l'étape **(e).**

La phase organique obtenue à l'étape **(e)** ou le mélange réactionnel obtenu à l'issue de l'étape **(c)** est ensuite concentré sous vide lors de l'étape **(f),** à une pression typiquement comprise entre 10 et 500 mbar, notamment entre 10 et 350 mbar, de préférence entre 10 et 150 mbar, en particulier entre 50 et 100 mbar, jusqu'à atteindre une température supérieure à 50°C, en particulier supérieure à 60°C, par exemple de 70°C, de façon à éliminer une partie du mélange ROH/$CH_3COOR$, puis séché sous vide, à une pression typiquement comprise entre notamment entre 10 et 350 mbar, de préférence entre 50 et 150 mbar, en particulier entre 50 et 100 mbar, jusqu'à atteindre une température supérieure à 50°C, en particulier supérieure à 60°C, par exemple de 70°C.

Cette étape peut être suivie d'une étape de filtration **(g),** qui peut être réalisée en utilisant toute méthode bien connue de l'homme du métier.

Le procédé selon l'invention permet d'obtenir le diester d'acide 2-bromoglutarique de formule (I) avec un rendement supérieur à 85 %, avantageusement supérieur à 90 %, et un degré de pureté, déterminé par analyse HPLC, supérieur ou égal à 90 %, en particulier supérieur ou égal à 93 %, typiquement supérieur ou égal à 95 %, de préférence supérieur ou égal à 97 %, notamment supérieur ou égal à 98 %, avantageusement supérieur ou égal à 99 %.

Selon un mode de réalisation préféré, R correspond à un groupement butyle, et le procédé selon l'invention permet de préparer le 2-bromoglutarate de dibutyle de formule (BGB) avec un rendement et un degré de pureté tels qu'énoncés précédemment.

La présente invention concerne donc, de surcroît, un diester d'acide 2-bromoglutarique de formule (I) suivante :

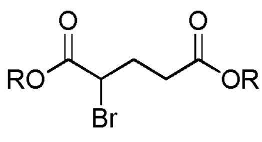

(I)

dans laquelle R représente un groupe ($C_3$-$C_6$)alkyle, de préférence un groupement butyle, ayant un degré de pureté, déterminé par analyse HPLC, supérieur ou égal à 90 %, en particulier supérieur ou égal à 93 %, typiquement supérieur ou égal à 95 %, de préférence supérieur ou égal à 97 %, notamment supérieur ou égal à 98%, avantageusement supérieur ou égal à 99 %.

## Etape de racémisation

Le diester d'acide 2-bromoglutarique de formule (I) peut être obtenu avec un excès énantiomérique variable. S'il n'est pas racémique, il est possible de le racémiser selon une étape additionnelle de racémisation en utilisant un sel de brome, tel que LiBr ou le bromure de tétrabutylammonium, selon des méthodes bien connues de l'homme du métier.

## FIGURES

La Figure 1 représente le spectre de masse du BGB.

## EXEMPLES

Les abréviations suivantes ont été utilisées :

| | |
|---|---|
| BGB | : 2-bromoglutarate de dibutyle |
| Bu | : butyle |
| ee | : excès énantiomérique |
| HPLC | : « High Performance Liquid Chromatography » |
| RMN | : résonance magnétique nucléaire |
| TBAB | : bromure de tétrabutylammonium |

## I. Synthèse du 2-bromoglutarate de dibutyle

### I.1. Protocole

147,1 g (1 mole) d'acide L-Glutamique sont dissous dans 294 g d'eau. Cette solution est chauffée à 55°C+/-5°C. On y ajoute lentement 76 g (1,1 mole) de nitrite de sodium en solution dans 152 g d'eau. Le contact est maintenu au moins 2 heures à 55°C+/-5°C jusqu'à solubilisation, puis la solution est refroidie à 20-25°C. Après neutralisation avec environ 122 g (1,1 mole) d'acide chlorhydrique à 33%, le milieu est concentré sous vide (< 100 mbar) en augmentant graduellement la température jusqu'à 60°C.

La butyrolactone acide obtenue est estérifiée avec 370 g (5 moles) de butanol en présence de 206 g d'acétate de butyle et 1,47 g (0,015 mole) d'acide sulfurique sous distillation azéotropique et sous vide. Le milieu réactionnel est ensuite refroidi à 0-5°C, repris avec 221 g d'eau. La phase aqueuse inférieure est retirée et la phase organique lavée 2 fois supplémentaires par 30 g d'eau. La phase organique obtenue contient un mélange d'hydroxyglutarate de butyle / ester butylique de la butyrolactone (HPLC s/s : 90/10). Le mélange est déshydraté par distillation azéotropique sous 50-100 mbar puis concentré en éliminant l'équivalent de 195 g de solvant.

La bromation est réalisée en suivant 5 fois les opérations successives suivantes : bullage de 100 g (1,2 mole) d'acide bromhydrique à 20+/-10°C, distillation azéotropique sous vide pendant au moins 5 heures. Le milieu réactionnel est lavé avec une solution aqueuse de 6 g d'hydrogénocarbonate de potassium dissous dans environ 60 g d'eau. La phase aqueuse est écartée et la phase organique est lavée à l'eau puis concentrée sous vide jusqu'à atteindre une température d'environ 70°C. Le BGB est obtenu avec un rendement de 90 % et une pureté déterminée par HPLC de 97,1 % s/s.

### I.2. Caractérisation du BGB

- Point d'ébullition 115-120 °C/0,03-0,04 kPa (0,2-0,3 mm Hg) soit environ 380°C à pression atmosphérique.
- RMN réalisée sur JEOL 500MHz :
  $^1$H RMN ($CDCl_3$, 400 MHZ) 4,34-4,39 (m, 1H, Br-CH-COO), 4,16-4,22 (m, 2H, Br-CH-$COOCH_2$), 4,06-4,11 (m, 2H, $CH_2$-$COOCH_2$), 2,50-2,59 (m, 2H, OOC-$CH_2$-$CH_2$-CHBr), 2,25-2,43 (m, 2H, OOC-$CH_2$-$CH_2$-CHBr), 1,55-1,69 (m, 4H, $CH_2$-$CH_2$-$CH_2$), 1,34-1,45 (m, 4H, $CH_3$-$CH_2$-CH2), 0,92-0,96 (m, 6H, $CH_3$-$CH_2$-$CH_2$)
- Spectrométrie de masse

La présence de brome dans la molécule a été confirmée par un spectre de masse réalisé à l'aide d'un spectromètre de masse Waters du type QDa couplé à un UHPLC Waters de type I-Class. L'enregistrement du spectre de masse a été réalisé en électrospray positif à la tension de cône de 10 V.

Le spectre de masse obtenu est représenté en Figure 1.

Les doublets avec une différence de 2 confirment la présence de brome dans la molécule.

La masse à 323-325 correspond au pic parent, et la masse à 249-251 à la perte du butanolate.

**I.3. Analyse HPLC**

• ***Matériel*** :

- Appareil HPLC constitué d'un système de pompage, un injecteur, une colonne chromatographique, un détecteur UV et d'une station de données.
- Colonne Spherisorb ODS 2, 250 x 4,6 mm - 5 μm.
- Acide sulfurique 96% Suprapur® (Merck 1.00714 ou équivalent).
- Acétonitrile (qualité HPLC gradient Grade, J.T Baker référence 8143 ou équivalent).
- Eau déionisée (qualité HPLC Elga, ou équivalent).

• ***Méthode*** :

- Phase mobile :

Voie A : 100% acétonitrile
Voie B : solution aqueuse d'acide sulfurique à 0,1 % v/v

- Préparation des échantillons :

Dans une fiole jaugée de 20 mL, introduire 0,2 g de BGB à analyser, puis une quantité suffisante d'acétonitrile pour atteindre 20 mL de solution.

- Conditions d'analyse :

| | |
|---|---|
| Température colonne | 25°C |
| Température échantillon | Ambiante |
| Débit | 1,0 mL/min |
| Volume d'injection | 20 μl |
| Détection UV | 220 nm |
| Durée d'analyse | 70 min |

- Gradient :

| Temps | % Acétonitrile | % $H_2SO_4$ (0,1 %) |
|---|---|---|
| 0 | 1 | 99 |
| 5 | 1 | 99 |
| 20 | 60 | 40 |
| 50 | 80 | 20 |
| 60 | 80 | 20 |
| 62 | 1 | 99 |
| 70 | 1 | 99 |

## II. Racémisation du 2-bromoglutarate de dibutyle

Lors de la synthèse, le carbone asymétrique conserve sa configuration et se racémise partiellement à la bromation. Le BGB est typiquement obtenu avec un excès énantiomérique variant de 50 à 80 %. Si le BGB obtenu n'est pas racémique, il est possible de le racémiser en utilisant un sel de brome.

### II.1. Avec LiBr

Racémisation des BGB avec 1 % molaire de LiBr par contact 2 h à 60°C.
Lavage du BGB racémique par 2 x 2 équivalents poids d'une solution de bicarbonate de sodium 0,1 M à 25°C.
Lavage avec 0,5 équivalent poids d'eau sous vide (pression ≤ 30 mbar) et température du milieu réactionnel à ≤ 70°C.
Ce procédé a été appliqué avec 3418 g de BGB et 9,22 g de LiBr avec un rendement de 97,9 %. Un suivi HPLC chirale a été réalisé pour atteindre la racémisation.

• ***Matériel*** :

- Appareil HPLC équipé d'un détecteur UV.
- Colonne Chiralpak IC - 5 µm - 250 x 4.6 mm de Daicel.

• ***Méthode*** :
- Phase mobile: 95 % heptane / 5 % alcool isopropylique
- Préparation des échantillons :
Dans une fiole jaugée de 10 mL, introduire 50 mg de BGB à analyser, puis une quantité suffisante d'heptane pour atteindre 10 mL de solution.
- Conditions d'analyse :
HPLC en phase normale, élution de la phase mobile en mode isocratique.

| | |
|---|---|
| Débit | 0,7 mL/min |
| Volume d'injection | 10 µl |
| Détection UV | 270 nm |

- Calcul de l'excès énantiomérique :

$$\% \text{ ee} = (\text{aire pic 1} - \text{aire pic 2}) / (\text{aire pic 1} + \text{aire pic 2}) * 100$$

| | % aire pic | | % excès énantiomérique |
|---|---|---|---|
| **Conditions** | **Enantiomère 1** | **Enantiomère 2** | |
| **Produit de départ** | 21,46 | 78,54 | -57,08 |
| **Contact 2h / 60°C** | 48,96 | 51,04 | -2,08 |
| **Contact 2h30 / 60 °C** | 49,68 | 50,32 | -0,64 |

### II.2. Avec TBAB

Il a été montré que la racémisation est également possible avec le TBAB dans les mêmes conditions qu'avec LiBr, sans solvant et à température ambiante.
3,2 g de TBAB sont ajoutés à 323 g de BGB. Le milieu est agité au moins 5h pour atteindre un ee < 1 %. Le TBAB est enlevé par 2 lavages successifs avec 150 ml d'eau. Le BGB est concentré sous vide à une température inférieure à 70°C.

## Revendications

**1.** Procédé de préparation du diester d'acide 2-bromoglutarique de formule (I) suivante :

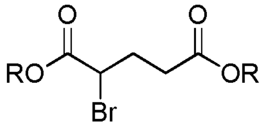

(I)

dans laquelle R représente un groupe ($C_3$-$C_6$)alkyle,
comprenant les étapes suivantes :
**(b)** formation du diester d'acide 2-hydroxyglutarique de formule (II)

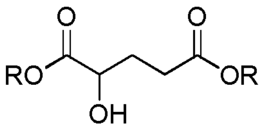

(II)

par réaction de l'acide butyrolactone de formule (BA)

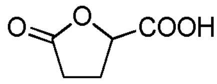

(BA)

avec l'alcool de formule ROH, en présence d'un acide tel que l'acide sulfurique ; et
**(c)** bromation du diester d'acide 2-hydroxyglutarique de formule (II), par bullage d'acide bromhydrique gazeux, conduisant au diester d'acide 2-bromoglutarique de formule (I).

**2.** Procédé selon la revendication 1, **caractérisé en ce que** l'étape **(b)** est réalisée en présence de l'acétate de formule $CH_3COOR$.

**3.** Procédé selon la revendication 1 ou 2, **caractérisé en ce qu'**au cours de l'étape **(b),** l'eau est éliminée par distillation sous vide.

**4.** Procédé selon l'une quelconque des revendication 1 à 3, **caractérisé en ce que** lors de l'étape **(b),** l'acide butyrolactone de formule (BA) et l'alcool ROH sont introduits dans des quantités telles que le rapport molaire ROH/BA est compris entre 2 et 10.

**5.** Procédé selon l'une quelconque des revendication 1 à 4, **caractérisé en ce qu'**à l'issue de l'étape **(b),** le milieu réactionnel est refroidi à une température inférieure à 15°C et est laissé à décanter après ajout d'eau de façon à obtenir la formation d'une phase organique et d'une phase aqueuse distinctes, ladite phase aqueuse étant ensuite éliminée et ladite phase organique étant déshydratée par distillation sous vide et/ou concentrée sous vide avant d'être soumise à l'étape **(c).**

**6.** Procédé selon l'une quelconque des revendications 1 à 5, **caractérisé en ce que** l'étape **(c)** comprend le cycle d'étapes suivantes :

- établissement de la température du milieu réactionnel à une valeur comprise entre 5°C et 40° C,
- introduction de l'acide bromhydrique gazeux dans une quantité comprise entre 1 et 1,5 éq. mol. par rapport à la quantité d'acide butyrolactone (BA) engagée dans l'étape **(b),**
- distillation sous vide pendant une durée avantageusement comprise entre 3h et 8h de façon à retirer de l'eau du milieu réactionnel,
- rétablissement de la température du milieu réactionnel à une valeur comprise entre 5°C et 40° C,

ledit cycle d'étapes étant avantageusement répété de 3 à 8 fois.

7. Procédé selon l'une quelconque des revendications 1 à 6, **caractérisé en ce qu'**il comprend une première étape **(a)** de formation de l'acide butyrolactone de formule (BA) par réaction de l'acide L-glutamique avec le nitrite de sodium en solution aqueuse.

8. Procédé selon l'une quelconque des revendications 1 à 7, **caractérisé en ce que** le mélange réactionnel obtenu à l'issue de l'étape **(c)** est soumis aux étapes additionnelles suivantes :

    **(d)** introduction du mélange réactionnel obtenu à l'issue de l'étape **(c)** dans une solution aqueuse basique, la solution obtenue ayant un pH typiquement compris entre 7,5 et 9,5 ;
    **(e)** décantation de la solution obtenue à l'étape **(d),** de façon à obtenir la formation d'une phase organique et d'une phase aqueuse distinctes, ladite phase aqueuse étant ensuite éliminée ;
    **(f)** concentration sous vide de la phase organique obtenue à l'étape **(e),** jusqu'à atteindre une température comprise entre 65 °C et 75°C, puis séchage sous vide;
    **(g)** optionnellement filtration ; et
    récupération du diester d'acide 2-bromoglutarique de formule (I).

9. Procédé selon l'une quelconque des revendications 1 à 8, **caractérisé en ce que** le diester d'acide 2-bromoglutarique de formule (I) obtenu à l'issue de l'étape **(c), (f)** ou **(g)** est soumis à une étape additionnelle de racémisation par ajout d'un sel de brome, tel que LiBr ou le bromure de tétrabutylammonium.

10. Procédé selon l'une quelconque des revendications 1 à 9, **caractérisé en ce que** R correspond à un groupement n-butyle.

11. Procédé selon l'une quelconque des revendications 3 à 10, **caractérisé en ce que** les opérations de distillation sous vide intervenant au cours des étapes **(b)** et **(c)** sont des distillations azéotropiques.

12. Composé de formule (I) suivante :

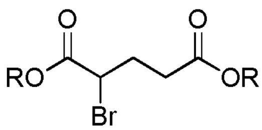

(I)

dans laquelle R représente un groupe ($C_3$-$C_6$)alkyle, de préférence un groupement butyle, ayant un degré de pureté, déterminé par analyse HPLC, supérieur ou égal à 90 %.

13. Composé selon la revendication 12, **caractérisé en ce que** R correspond à un groupement n-butyle.

14. Composé selon la revendication 12 ou 13, **caractérisé en ce que** son degré de pureté est supérieur ou égal à 95 %.

15. Composé selon l'une quelconque des revendications 12 à 14, **caractérisé en ce que** son degré de pureté est supérieur ou égal à 97 %.

## Patentansprüche

1. Verfahren zur Herstellung des 2-Bromglutarsäurediesters der folgenden Formel (I):

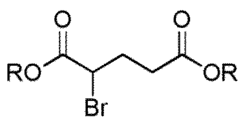

(I)

in der R für eine ($C_3$-$C_6$)Alkylgruppe steht,
umfassend die folgenden Schritte:
**(b)** Bilden des 2-Hydroxyglutarsäurediesters der Formel (II)

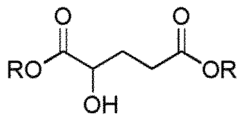

(II)

durch Umsetzung von Butyrolactonsäure der Formel (BA)

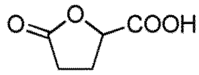

(BA)

mit dem Alkohol der Formel ROH in Gegenwart einer Säure wie Schwefelsäure; und

**(c)** Bromieren des 2-Hydroxyglutarsäurediesters der Formel (II) durch Durchleiten von gasförmiger Bromwasserstoffsäure, was den 2-Bromglutarsäurediester der Formel (I) ergibt.

2. Verfahren nach Anspruch 1, **dadurch gekennzeichnet, dass** Schritt **(b)** in Gegenwart des Essigsäureesters der Formel $CH_3COOR$ durchgeführt wird.

3. Verfahren nach Anspruch 1 oder 2, **dadurch gekennzeichnet, dass** im Verlauf von Schritt **(b)** das Wasser durch Vakuumdestillation entfernt wird.

4. Verfahren nach einem der Ansprüche 1 bis 3, **dadurch gekennzeichnet, dass** im Verlauf von Schritt **(b)** die Butyrolactonsäure der Formel (BA) und der Alkohol ROH in solchen Mengen eingetragen werden, dass das ROH/BA-Molverhältnis zwischen 2 und 10 liegt.

5. Verfahren nach einem der Ansprüche 1 bis 4, **dadurch gekennzeichnet, dass** am Ende von Schritt **(b)** das Reaktionsmedium auf eine Temperatur unterhalb von 15 °C abgekühlt und nach Zugabe von Wasser absetzen gelassen wird, wodurch eine organische Phase und eine separate wässrige Phase erhalten werden, wobei die wässrige Phase dann entfernt wird und die organische Phase durch Vakuumdestillation entwässert und/oder unter Vakuum aufkonzentriert wird, bevor sie dem Schritt **(c)** unterworfen wird.

6. Verfahren nach einem der Ansprüche 1 bis 5, **dadurch gekennzeichnet, dass** Schritt **(c)** den Zyklus der folgenden Schritte umfasst:

   - Einstellen der Temperatur des Reaktionsmediums auf einen Wert zwischen 5 °C und 40 °C,
   - Eintragen von gasförmiger Bromwasserstoffsäure in einer Menge zwischen 1 und 1,5 Mol. Äq., bezogen auf die in Schritt (b) verwendete Menge an Butyrolactonsäure (BA),
   - Vakuumdestillation über einen Zeitraum, der vorteilhafterweise zwischen 3 h und 8 h liegt, zum Abziehen von Wasser aus dem Reaktionsmedium,
   - Wiedereinstellen der Temperatur des Reaktionsmediums auf einen Wert zwischen 5 °C und 40 °C,

   wobei der Zyklus von Schritten vorteilhafterweise 3- bis 8-mal wiederholt wird.

7. Verfahren nach einem der Ansprüche 1 bis 6, **dadurch gekennzeichnet, dass** es einen ersten Schritt **(a)** der Bildung von Butyrolactonsäure der Formel (BA) durch Umsetzung von L-Glutaminsäure mit Natriumnitrit in wässriger Lösung umfasst.

8. Verfahren nach einem der Ansprüche 1 bis 7, **dadurch gekennzeichnet, dass** die am Ende von Schritt **(c)** erhaltene Reaktionsmischung den folgenden zusätzlichen Schritten unterworfen wird:

   **(d)** Eintragen der am Ende von Schritt **(c)** erhaltenen Reaktionsmischung in eine basische wässrige Lösung, wobei die erhaltene Lösung typischerweise einen pH-Wert zwischen 7,5 und 9,5 aufweist;
   **(e)** Absetzenlassen der aus Schritt **(d)** erhaltenen Lösung, wodurch eine organische Phase und eine separate wässrige Phase erhalten werden, wobei die wässrige Phase dann entfernt wird;
   **(f)** Aufkonzentrieren der in Schritt (e) erhaltenen organischen Phase bis zum Erreichen einer Temperatur zwischen 65 °C und 75 °C und dann Trocknen unter Vakuum;
   **(g)** gegebenenfalls Filtration; und

Gewinnung des 2-Bromglutarsäurediesters der Formel (I).

**9.** Verfahren nach einem der Ansprüche 1 bis 8, **dadurch gekennzeichnet, dass** der am Ende von Schritt **(c), (f)** oder **(g)** erhaltene 2-Bromglutarsäurediester der Formel (I) einem zusätzlichen Racemisierungsschritt durch Zugabe eines Bromsalzes, wie LiBr oder Tetrabutylammoniumbromid, unterworfen wird.

**10.** Verfahren nach einem der Ansprüche 1 bis 9, **dadurch gekennzeichnet, dass** R einer n-Butylgruppe entspricht.

**11.** Verfahren nach einem der Ansprüche 3 bis 10, **dadurch gekennzeichnet, dass** es sich bei den im Verlauf der Schritte **(b)** und **(c)** stattfindenden Vakuumdestillationsarbeitsgängen um azeotrope Destillationen handelt.

**12.** Verbindung der folgenden Formel (I):

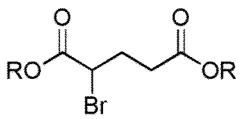
(I)

in der R für eine ($C_3$-$C_6$)Alkylgruppe , vorzugsweise eine Butylgruppe, steht, mit einem durch HPLC-Analyse bestimmten Reinheitsgrad größer oder gleich 90 %.

**13.** Verbindung nach Anspruch 12, **dadurch gekennzeichnet, dass** R einer n-Butylgruppe entspricht.

**14.** Verbindung nach Anspruch 12 oder 13, **dadurch gekennzeichnet, dass** ihr Reinheitsgrad größer oder gleich 95 % ist.

**15.** Verbindung nach einem der Ansprüche 12 bis 14, **dadurch gekennzeichnet, dass** ihr Reinheitsgrad größer oder gleich 97 % ist.

**Claims**

**1.** Process for preparing a 2-bromoglutaric acid diester of formula (I) below:

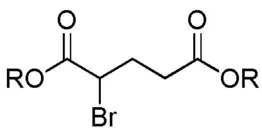
(I)

in which R represents a ($C_3$-$C_6$)alkyl group,
comprising the following steps:
**(b)** forming the 2-hydroxyglutaric acid diester of formula (II)

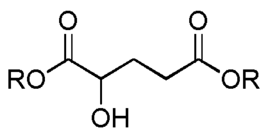
(II)

by reacting the butyrolactone acid of formula (BA)

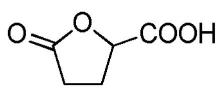
(BA)

with the alcohol of formula ROH, in the presence of an acid such as sulfuric acid; and

**(c)** brominating the 2-hydroxyglutaric acid diester of formula (II), by sparging with gaseous hydrobromic acid, leading to the 2-bromoglutaric acid diester of formula (I) .

**2.** Process according to Claim 1, **characterized in that** step **(b)** is performed in the presence of the acetate of formula $CH_3COOR$.

**3.** Process according to Claim 1 or 2, **characterized in that**, during step **(b),** water is removed by vacuum distillation.

**4.** Process according to any one of Claims 1 to 3, **characterized in that** in step **(b),** the butyrolactone acid of formula (BA) and the alcohol ROH are introduced in amounts such that the ROH/BA mole ratio is between 2 and 10.

**5.** Process according to any one of Claims 1 to 4, **characterized in that**, on conclusion of step **(b),** the reaction medium is cooled to a temperature below 15°C and is left to settle after addition of water so as to obtain the formation of an organic phase and a separate aqueous phase, said aqueous phase then being removed and said organic phase being dehydrated by vacuum distillation and/or concentration under vacuum before being subjected to step **(c).**

**6.** Process according to any one of Claims 1 to 5, **characterized in that** step **(c)** comprises the following cycle of steps:

- setting the temperature of the reaction medium to a value of between 5°C and 40°C,
- introducing gaseous hydrobromic acid in an amount of between 1 and 1.5 mol. eq. relative to the amount of butyrolactone acid (BA) used in step **(b),**
- vacuum distillation for a time advantageously between 3h and 8h so as to remove water from the reaction medium,
- restoring the temperature of the reaction medium to a value of between 5°C and 40°C,

said cycle of steps being advantageously repeated from 3 to 8 times.

**7.** Process according to any one of Claims 1 to 6, **characterized in that** it comprises a first step **(a)** of forming the butyrolactone acid of formula (BA) by reacting L-glutamic acid with sodium nitrite in aqueous solution.

**8.** Process according to any one of Claims 1 to 7, **characterized in that** the reaction mixture obtained on conclusion of step **(c)** is subjected to the following additional steps:

**(d)** introducing the reaction mixture obtained on conclusion of step **(c)** into a basic aqueous solution, the solution obtained having a pH typically between 7.5 and 9.5;
**(e)** separating by settling the solution obtained in step **(d),** so as to obtain the formation of an organic phase and a separate aqueous phase, said aqueous phase being then removed;
**(f)** concentration under vacuum of the organic phase obtained in step **(e),** until a temperature of between 65°C and 75°C is reached, followed by drying under vacuum;
**(g)** optionally, filtration; and
recovering the 2-bromoglutaric acid diester of formula (I) .

**9.** Process according to any one of Claims 1 to 8, **characterized in that** the 2-bromoglutaric acid diester of formula (I) obtained on conclusion of step **(c), (f)** or **(g)** is subjected to an additional racemization step by adding a bromine salt, such as LiBr or tetrabutylammonium bromide.

**10.** Process according to any one of Claims 1 to 9, **characterized in that** R corresponds to an n-butyl group.

**11.** Process according to any one of Claims 3 to 10, **characterized in that** the vacuum distillation operations taking place during steps **(b)** and **(c)** are azeotropic distillations.

**12.** A compound of formula (I) below:

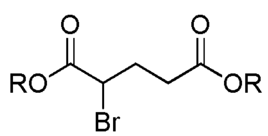

(I)

in which R represents a $(C_3-C_6)$alkyl group, preferably a butyl group,
with a degree of purity, determined by HPLC analysis, of greater than or equal to 90%.

**13.** Compound according to Claim 12, **characterized in that** R corresponds to an n-butyl group.

**14.** Compound according to Claim 12 or 13, **characterized in that** its degree of purity is greater than or equal to 95%.

**15.** Compound according to any one of Claims 12 to 14, **characterized in that** its degree of purity is greater than or equal to 97%.

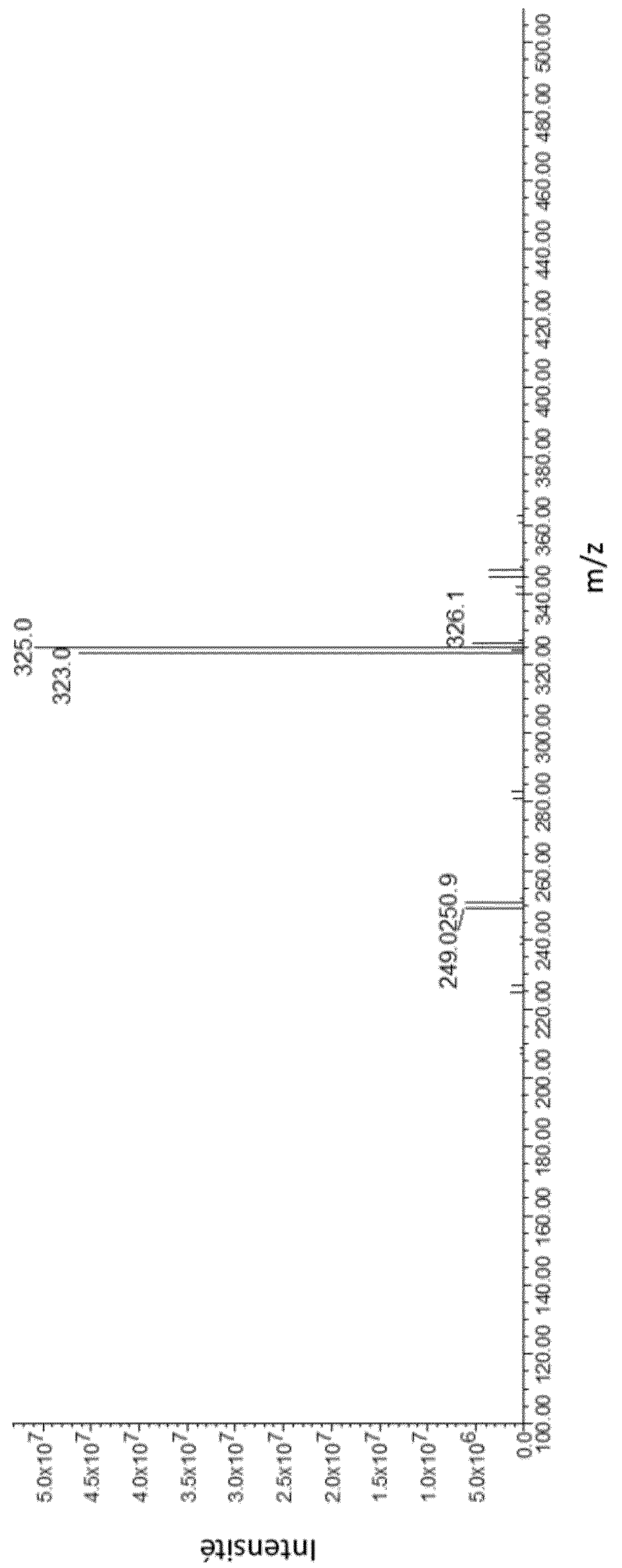
325.0
323.0
326.1
249.0
250.9
m/z
Intensité
5.0x10^7
4.5x10^7
4.0x10^7
3.5x10^7
3.0x10^7
2.5x10^7
2.0x10^7
1.5x10^7
1.0x10^7
5.0x10^6
0.0
100.00
120.00
140.00
160.00
180.00
200.00
220.00
240.00
260.00
280.00
300.00
320.00
340.00
360.00
380.00
400.00
420.00
440.00
460.00
480.00
500.00

**Fig. 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1931673 A
- CS 209266 B1
- GB 577877 A


### Littérature non-brevet citée dans la description


- *Chemistry a European Journal,* vol. 16 (35), 10727-10734
- *CHEMICAL ABSTRACTS,* 104867-13-2